# EUROPEAN PATENT APPLICATION

(11) **EP 4 442 280 A1**
(43) Date of publication of application: **09.10.2024**
(21) Application number: 22901407.1
(22) Date of filing: 01.12.2022
(51) Int. Cl.: A61K 45/00, A61K 39/02, A61K 39/395, A61P 37/08, C12Q 1/02, G01N 33/68, C07K 14/195

(54) **ESTABLISHMENT OF THERAPY AND DIAGNOSIS FOR ALLERGIC DISEASES THROUGH CONTROL OF IMMUNOGLOBULIN-BINDING PROTEIN**

(30) Priority: 02.12.2021 JP 2021195941
(71) Applicant: KANAGAWA INSTITUTE OF INDUSTRIAL SCIENCE AND TECHNOLOGY, Ebina-shi Kanagawa 243-0435 (JP); University of Tsukuba, Ibaraki 305-8577 (JP); Metagen, Inc., Tsuruoka-shi, Yamagata 997-0052 (JP)
(72) Inventor: Fukuda Shinji, Tsuruoka-shi, Yamagata 997-0052 (JP); Nakato Gaku, Ebina-shi, Kanagawa 243-0435 (JP); OBANA Nozomu, Tsukuba-shi, Ibaraki 305-8577 (JP); Furukawa Risako, Tsuruoka-shi, Yamagata 997-0052 (JP)
(74) Representative: Plasseraud IP
(86) International application number: PCT/JP2022/044364
(87) International publication number: WO 2023/100977

(57) **Abstract**

The present invention addresses the problem of revealing the mechanism of allergy development in which a bacterium of the family *Lachnospiraceae,* which is an enteric bacterium, including *Ruminococcus gnavus* (*R. gnavus*) is involved and of developing a means for preventing or treating allergies by intervening in the mechanism. The inventors of the present invention conducted an intensive study and, as a result, found that an Ibp protein from a bacterium of the family *Lachnospiraceae* is involved in development of allergies and showed that the above problem can be solved. That is, it is possible to prevent allergic reactions occurring in vivo by administration or induction of in vivo production of a substance that binds to the Ibp protein from a bacterium of the family *Lachnospiraceae.*

## Description

### Technical Field

The present invention relates to providing a composition for prevention and treatment of allergies.

### Background Art

In an allergy, when an allergy-causing substance, allergen (antigen), derived from food, pollen, dust mites, or the like enters the living body, IgE antibodies are produced (sensitization), and subsequently, when the allergen enters the body again, the IgE antibodies react to the allergen to release chemical mediators such as histamine from mast cells to cause allergy symptoms.

There are still a lot of unknown onset mechanism of an allergy at present, but in recent years, the relationship with the intestinal microbiota has attracted attention. It has been reported that, in studies in pediatric allergy patients, *Ruminococcus gnavus* (hereinafter, referred to as *"R. gnavus*") increases in the intestines, and it has also been reported that the administration of *R. gnavus* to mice is associated with and promotes the development of allergies, in particular, respiratory allergies (Non Patent Literature 1).

Regarding the bacteria *R. gnavus,* IgA binding properties was examined, and it has been reported that immunoglobulin-binding proteins (Ibps), IbpA and IbpB, of *R. gnavus* bind to human or mouse IgA as superantigens and that these superantigens act on B cells to increase IgA production (Non Patent Literature 2) .

Ibp proteins are present in various bacterial species including *R. gnavus,* and two homologous proteins, IbpA (66 kDa protein) and IbpB (70 kDa protein), are present in *R. gnavus.* As a result of analysis of amino acid structure, these proteins comprise a secretion signal sequence and a cellular localization motif having a peptide sequence of LPXTG. Accordingly, it is considered to function as cell wall proteins.

However, it is unclear through what mechanism of action *R*. *gnavus* is involved in the development of allergies and whether two homologous proteins, IbpA and IbpB, are involved in the mechanism of action.

### Citation List

### Non Patent Literature

Non Patent Literature 1: Chua, et al., Gastroenterology, 154: 154-167, 2018
Non Patent Literature 2: Bunker, et al., Sci. Transl. Med., 11, eaau9356, 2019

### Summary of Invention

### Technical Problem

The present invention addresses the problem of revealing the mechanism of allergy development in which a bacterium of the family *Lachnospiraceae,* is an enteric bacterium, including *Ruminococcus gnavus* (*R. gnavus*), is involved and of developing a means for preventing or treating allergies by intervening in the mechanism.

### Solution to Problem

The inventors of the present invention conducted an intensive study and, as a result, found that an Ibp protein from a bacterium of the family *Lachnospiraceae* is involved in development of allergies and showed that the above problems can be solved. That is, it is possible to prevent allergic reactions occurring in vivo by administration or induction of in vivo production of a substance that binds to the Ibp protein from a bacterium of the family *Lachnospiraceae.*

More specifically, in order to solve the above-mentioned problems, the present application provides the following embodiments:
[1] A pharmaceutical composition for treating or preventing an allergy, comprising a substance that binds to an Ibp protein of a bacterium of the family *Lachnospiraceae* or its variant;
[2] The pharmaceutical composition according to [1], wherein the Ibp protein or its variant is IbpA protein (SEQ ID NO: 1) or IbpB protein (SEQ ID NO: 2), or a protein having an amino acid sequence comprising substitution, insertion, or deletion of one or several amino acids in these amino acid sequences; or a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB), or a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB);
[3] The pharmaceutical composition according to [1] or [2], wherein the substance that binds to an Ibp protein of a bacterium of the family *Lachnospiraceae* or its variant is an antibody or an antibody derivative;
[4] The pharmaceutical composition according to [1] or [2], wherein the substance that binds to an Ibp protein of a bacterium of the family *Lachnospiraceae* or its variant is IgG, IgM, or IgA;
[5] The pharmaceutical composition according to [1] or [2], wherein the bacterium of the family *Lachnospiraceae* is a bacterium of genus *Ruminococcus;*
[6] The pharmaceutical composition according to [5], wherein the bacterium of genus *Ruminococcus* is *Ruminococcus gnavus (R. gnavus*);
[7] The pharmaceutical composition according to [1] or [2], wherein the substance that binds to an Ibp protein of a bacterium of the family *Lachnospiraceae* or its variant binds to a protein of a repeat region comprising the amino acid sequence of amino acid Nos. 111 to 378 of SEQ ID NO: 1 or amino acid Nos. 126 to 393 of SEQ ID NO: 2, a protein of C-terminal region comprising the amino acid sequence of amino acid Nos. 379 to 574 of SEQ ID NO: 1 or amino acid Nos. 394 to 592 of SEQ ID NO: 2, or a protein having an amino acid sequence comprising substitution, insertion, or deletion of one or several amino acids in the amino acid sequence of the repeat region or C-terminal region; or a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB), or a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB);
[8] A vaccine composition for preventing or treating an allergy, comprising the full-length or a portion of an Ibp protein of a bacterium of the family *Lachnospiraceae* or its variant as an immunogen;
[9] The vaccine composition according to [8], wherein the Ibp protein or its variant is IbpA protein (SEQ ID NO: 1) or IbpB protein (SEQ ID NO: 2), or a protein having an amino acid sequence comprising substitution, insertion, or deletion of one or several amino acids in these amino acid sequences; a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB), or a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB);
[10] The vaccine composition according to [8] or [9], wherein the bacterium of the family *Lachnospiraceae* is a bacterium of genus *Ruminococcus*;
[11] The vaccine composition according to [10], wherein the bacterium of genus *Ruminococcus* is *Ruminococcus gnavus (R. gnavus*);
[12] The vaccine composition according to [8] or [9], wherein the portion of the Ibp protein or its variant is a protein of a repeat region comprising the amino acid sequence of amino acid Nos. 111 to 378 of SEQ ID NO: 1 or amino acid Nos. 126 to 393 of SEQ ID NO: 2, a protein of C-terminal region comprising the amino acid sequence of amino acid Nos. 379 to 574 of SEQ ID NO: 1 or amino acid Nos. 394 to 592 of SEQ ID NO: 2, or a protein having an amino acid sequence comprising substitution, insertion, or deletion of one or several amino acids in the amino acid sequence of the repeat region or C-terminal region; or a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB), or a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB);
[13] The vaccine composition according to [8] or [9], wherein when the vaccine composition is administered, IgG, IgM, or IgA is produced in vivo;
[14] The vaccine composition according to [8] or [9], wherein IgG, IgM, or IgA that binds to the Ibp protein of a bacterium of the family *Lachnospiraceae* or its variant is produced in a human body to which the vaccine composition is administered;
[15] The vaccine composition according to [8] or [9], wherein the vaccine composition is administered by subcutaneous, intradermal, intramuscular, intravenous, or mucosal membrane administration;
[16] A method for detecting the presence of a predisposition to developing an allergy in a subject, comprising:
   (1) a step of detecting the Ibp protein or its variant in a living body-derived sample collected from a subject; and
   (2) a step of associating the detection of the Ibp protein or its variant in the above (1) with the subject having a predisposition to developing an allergy;
[17] The method according to [16], wherein the living body-derived sample is a blood sample or a fecal sample;
[18] The method according to [16] or [17], wherein the Ibp protein or its variant is IbpA protein (SEQ ID NO: 1) or IbpB protein (SEQ ID NO: 2), or a protein having an amino acid sequence comprising substitution, insertion, or deletion of one or several amino acids in these amino acid sequences; or a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB), or a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB);
[19] A method for detecting the presence of a predisposition to developing an allergy in the serum derived from a subject, comprising:
   (1) a step of culturing human mast cells in the presence of the serum derived from a subject and measuring the amount of histamine in a culture supernatant;
   (2) a step of adding and reacting IgG, IgM, or IgA to the serum from the subject, and then culturing human mast cells in the presence of the serum and measuring the amount of histamine in a culture supernatant; and
   (3) a step of associating the amount of histamine obtained in the above (1) being higher than the amount of histamine obtained in the above (2) with the presence of a predisposition to developing an allergy in the blood of the subject;
[20] A method for selecting an inhibitor against functional relations between IgE and an Ibp protein of a bacterium of the family *Lachnospiraceae* or its variant, comprising:
   (1) a step of culturing mast cells in the presence of a test substance under conditions containing IgE and an Ibp protein of a bacterium of the family *Lachnospiraceae* or its variant and measuring the amount of histamine in a culture supernatant;
   (2) a step of culturing mast cells in absence of the test substance under conditions containing IgE and the Ibp protein of a bacterium of the family *Lachnospiraceae* or its variant and measuring the amount of histamine in a culture supernatant; and
   (3) a step of selecting the test substance as a substance having activity of inhibiting functional relations between IgE and the Ibp protein of a bacterium of the family *Lachnospiraceae* or its variant, when the amount of histamine measured in the above (1) is lower than the amount of histamine measured in the above (2);
[21] The method according to [20], wherein the Ibp protein or its variant is IbpA protein (SEQ ID NO: 1) or IbpB protein (SEQ ID NO: 2), or a protein having an amino acid sequence comprising substitution, insertion, or deletion of one or several amino acids in these amino acid sequences; or a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB), or a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB);
[22] The method according to [20] or [21], wherein the bacterium of the family *Lachnospiraceae* is a bacterium of genus *Ruminococcus;* and
[23] The method according to [22], wherein the bacterium of genus *Ruminococcus* is *Ruminococcus gnavus* (*R. gnavus*)*.*

### Advantageous Effects of Invention

It was found that an Ibp protein from a bacterium of the family *Lachnospiraceae* is involved in development of allergies, and as a result, the present invention can provide a means for preventing or treating allergic reaction in vivo by administering a substance that binds to the Ibp protein of a bacterium of the family *Lachnospiraceae* (for example, a bacterium of genus *Ruminococcus*) or its variant to a living body. The present invention can also provide a vaccine composition containing the whole or a portion of the Ibp protein or its variant as an immunogen, wherein the vaccine composition is able to produce an antibody that binds to the Ibp protein or its variant in vivo upon administration to a living body.

### Brief Description of Drawings

[Figure 1] Figure 1 shows the results of Western blotting for investigating intrabacterial localization of an Ibp protein using fraction samples from *R. gnavus* cells or fraction samples from Ibp protein-deficient *R. gnavus* cells.
[Figure 2] Figure 2 is a diagram showing the flow of an experiment for quantitative measurement of the amount of histamine released from the activated mast cells into a culture supernatant using human mast cell strain LAD2 cells.
[Figure 3] Figure 3 is a graph showing that histamine secretion is induced in LAD2 cells by sensitization with hIgE and stimulation with an Ibp protein.
[Figure 4] Figure 4 is a graph showing that histamine secretion is not induced in LAD2 cells sensitized with hIgE by stimulation with an Ibp protein that has been pre-reacted with IgG.
[Figure 5] Figure 5 is a diagram schematically showing various fragments of an Ibp protein, which were produced for investigating the binding mode between the Ibp protein and IgE.
[Figure 6] Figure 6 shows that high affinity binding of an Ibp protein to IgG and IgA requires both the repeat region and the C-terminal region of the Ibp protein, but the C-terminal region of the Ibp protein plays a major role in binding of the Ibp protein to IgE.

### Description of Embodiments

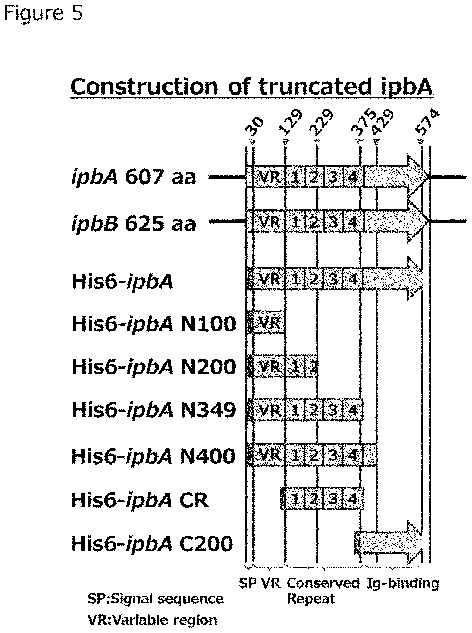

The Ibp protein is an immunoglobulin binding protein found in *R. gnavus,* and the Ibp protein of *R. gnavus* includes homologs IbpA and IbpB. IbpA of *R. gnavus* has 607 amino acids (WP_105084811.1, SEQ ID NO: 1), IbpB has 625 amino acids (WP_105084812.1, SEQ ID NO: 2), and both proteins have a structure in which, from the N-terminal side, a signal peptide (SP), a variable region (VR), a conservative portion of a repeat sequence, and an Ig-binding site are aligned (see Figure 5). It was known that the Ibp protein has an ability to nonspecifically bind to mouse IgG, IgM, and IgA by the Ig-binding site present in the C-terminal region of the Ibp protein.

The Ibp protein is present also in other bacteria belonging to the family *Lachnospiraceae.* Regarding IbpA, as proteins having homology to the amino acid sequence from the conservative portion of the repeat sequence to the C-terminus (amino acid Nos. 111 to 607 of SEQ ID NO: 1), the following proteins have been registered:
*Lachnospiraceae* bacterium TM07-2AC (Accession No. RJW20905.1); *Ruminococcus* sp. (Accession No. MBS4908879.1); and
*Lachnospiraceae* bacterium EP-SM-12S-S03 (Accession No. MCB5880945.1). As proteins having homology to the amino acid sequence of the C-terminal region comprising the Ig-binding site (amino acid Nos. 379 to 607 of SEQ ID NO: 1), the following proteins have been registered:
   *Lachnospiraceae* bacterium 2_1_58FAA (Accession No. EGN43539.1); *Ruminococcus* sp. (Accession No. SCI97089.1);
   *Lachnospiraceae* bacterium 2_1_58FAA (Accession No. EGN43802.1); *Ruminococcus* sp. (Accession No. HBJ44652.1);
   *Lachnospiraceae* bacterium TM07-2AC (Accession No. RJW20905.1); *Ruminococcus* sp. (Accession No. MBS4908879.1); and
   *Lachnospiraceae* bacterium (Accession No. MBS6939147.1). It is considered that these proteins also have the same function as that of the Ibp protein from *R. gnavus.*

Similarly, regarding IbpB, as proteins having homology to the amino acid sequence from the conservative portion of the repeat sequence to the C-terminus (amino acid Nos. 126 to 625 of SEQ ID NO: 2), the following protein has been registered: *Lachnospiraceae* bacterium TM07-2AC (Accession No. RJW20905.1). As proteins having homology to the amino acid sequence of the C-terminal region comprising the Ig-binding site (amino acid Nos. 394 to 625 of SEQ ID NO: 2), the following proteins have been registered:
*Lachnospiraceae* bacterium 2_1_58FAA (Accession No. EGN43802.1); *Ruminococcus* sp. (Accession No. HBJ44652.1);
*Lachnospiraceae* bacterium TM07-2AC (Accession No. RJW20905.1); *Lachnospiraceae* bacterium 2_1_58FAA (Accession No. EGN43539.1); Uncultured *Ruminococcus* sp. (Accession No. SCI97089.1);
*Ruminococcus* sp. (Accession No. MBS4908879.1); and *Lachnospiraceae* bacterium (Accession No. MBS6939147.1). It is considered that these proteins also have the same function as that of the Ibp protein from *R. gnavus.*

The inventors of the present invention found for the first time in the art that the Ibp protein of a bacterium of the family *Lachnospiraceae* nonspecifically binds to IgE in a human living body as the mechanism of allergy development in which the bacterium of the family *Lachnospiraceae,* which is an enteric bacterium, including *Ruminococcus gnavus* (*R. gnavus*), is involved and, therefore, revealed that the Ibp protein enhances the histamine production from mast cells and, as a result, causes allergic reaction.

In addition, the inventors of the present invention also revealed that allergic reaction in vivo can be prevented by administering a substance that binds to an Ibp protein of a bacterium of the family *Lachnospiraceae* or its variant to a living body to inhibit and intervene in the binding between the Ibp protein or its variant and IgE in vivo, which is a part of the above-described mechanism of development of allergic reaction.

Based on the above findings, the present invention can provide, as a first embodiment, a pharmaceutical composition for treating or preventing an allergy, comprising a substance that binds to an Ibp protein of a bacterium of the family *Lachnospiraceae* or its variant.

The substance as an active ingredient of the pharmaceutical composition of this embodiment binds to the Ibp protein of a bacterium of the family *Lachnospiraceae* or its variant and, as a result, can inhibit the nonspecific binding between the Ibp protein of a bacterium of the family *Lachnospiraceae* or its variant and IgE in vivo. The substance as the active ingredient may be any substance as long as it can inhibit the nonspecific binding between the Ibp protein or its variant and IgE. The Ibp protein of a bacterium of the family *Lachnospiraceae* or its variant binds to IgE in vivo and thereby enhances histamine production from mast cells. On the other hand, the substance can inhibit allergy development by inhibiting nonspecific binding between the Ibp protein of a bacterium of the family *Lachnospiraceae* or its variant and IgE and thereby inhibiting histamine production from mast cells.

In the present invention, the term "Ibp protein or its variant" includes, in addition to IbpA protein of *R. gnavus* (SEQ ID NO: 1) and IbpB protein of *R. gnavus* (SEQ ID NO: 2), a protein having an amino acid sequence comprising substitution, insertion, or deletion of one or several amino acids in these amino acid sequences, a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB), and a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB).

Here, in the phrase "a protein having an amino acid sequence comprising substitution, insertion, or deletion of one or several amino acids in these amino acid sequences", "one or several" amino acids means, for example, the number of amino acid residues in the range of 1 to 50, and is preferably 1 to 20, more preferably 1 to 10, and further preferably 1 to 5, although the range varies depending on the position and type of each amino acid residue in the three-dimensional structure of IbpA protein or IbpB protein of *R. gnavus.* In the phrase, "substitution, insertion, or deletion of amino acids" means a mutation which maintains the characteristics of the Ibp protein in the present invention and, specifically, may be any mutation as long as it causes nonspecific binding with IgE.

In the phrase "a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB) ", the variant of the Ibp protein may be a protein comprising an amino acid sequence having, for example, 50% or more, 65% or more, 80% or more, preferably 90% or more, more preferably 95% or more, further preferably 97% or more, and particularly preferably 99% or more identity with the amino acid sequence from the conservative portion of the repeat sequence to the C-terminus of the Ibp protein (amino acid Nos. 111 to 607 of SEQ ID NO: 1 or amino acid Nos. 126 to 625 of SEQ ID NO: 2), as long as it causes nonspecific binding with IgE as in the Ibp protein.

In the phrase "a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB) ", the variant of the Ibp protein may be a protein comprising an amino acid sequence having, for example, 80% or more, preferably 90% or more, more preferably 95% or more, further preferably 97% or more, and particularly preferably 99% or more identity with the amino acid sequence of the C-terminal region comprising the Ig-binding site of the Ibp protein (amino acid Nos. 379 to 607 of SEQ ID NO: 1 or amino acid Nos. 394 to 625 of SEQ ID NO: 2), as long as it causes nonspecific binding with IgE as in the Ibp protein.

It has been revealed that the Ibp protein or its variant of the present invention include the above-described proteins for IbpA and IbpB, respectively, and, specifically, it has been revealed in the present application that these variants of the Ibp proteins include, regarding IbpA, 52.12% to 99.4% amino acid sequence identity with the amino acid sequence from the conservative portion of the repeat sequence to the C-terminus (amino acid Nos. 111 to 607 of SEQ ID NO: 1) or 85.41% to 100% amino acid sequence identity with the amino acid sequence of the C-terminal region comprising the Ig-binding site (amino acid Nos. 379 to 607 of SEQ ID NO: 1), and include, regarding IbpB, 99.4% amino acid sequence identity with the amino acid sequence from the conservative portion of the repeat sequence to the C-terminus (amino acid Nos. 126 to 625 of SEQ ID NO: 2) or 85.41% to 100% amino acid sequence identity with the amino acid sequence of the C-terminal region comprising the Ig-binding site (amino acid Nos. 394 to 625 of SEQ ID NO: 2).

The substance as the active ingredient in the pharmaceutical composition of the present invention may be, but not limited to, for example, a synthetic compound such as a low-molecular compound and a medium-molecular compound or a high-molecular compound such as an antibody. For example, an antibody that binds to the Ibp protein from a bacterium of genus *Ruminococcus* has been generated in Example, and, therefore, it has been confirmed in the specification that an antibody or its derivative can be used as an active ingredient.

In the case where the substance as the active ingredient in the pharmaceutical composition of the present invention is an antibody, the antibody is an antibody other than IgE. The present invention can only use antibodies other than IgE since it has been shown that IgE and the Ibp protein of a bacterium of the family *Lachnospiraceae* or its variant are nonspecifically bound in vivo to cause an allergy, as a result. Accordingly, IgE cannot be used as a substance that is an active ingredient. That is, in the case where the active ingredient in the pharmaceutical composition of the present invention is an antibody, IgG, IgA, IgM, or IgD can be used, and IgG, IgM, or IgA is preferably used.

Since the Ibp protein has an ability to nonspecifically bind to antibodies, as a superantigen, the antibody as a substance that is an active ingredient here may be an antibody that specifically binds to the Ibp protein (an antibody that specifically binds to a portion of the Ibp protein as an epitope) or may be an antibody that nonspecifically binds to the Ibp protein (for example, an isotype control antibody).

As the substance that is the active ingredient in the pharmaceutical composition of the present invention, the derivatives of these antibodies can also be used, as long as the derivatives can bind to the Ibp protein of a bacterium of the family *Lachnospiraceae* or its variant and can inhibit the binding between the Ibp protein or its variant and IgE. The antibody derivative that can be used in the present invention may include, but not limited to, for example, a human-type antibody variant selected from a single strand antibody (such as scFv), a humanized antibody, a chimeric antibody, a multivalent antibody, and a multispecific antibody, or the functional fragment thereof. Among them, as a functional fragment, for example, but not limited to, F(ab')2 can be used.

In the present invention, it was possible to confirm the mechanisms between bacteria of the family *Lachnospiraceae* including *R. gnavus* and allergies, which had not been confirmed in the prior art (for example, Non Patent Literature 1). Based on this findings, the target of the pharmaceutical composition of the present invention is the Ibp protein of bacteria of the family *Lachnospiraceae* or its variant that binds to IgE and causes allergies, more specifically, the target is the Ibp protein or its variant derived from bacteria of the genus *Ruminococcus* belonging to the family *Lachnospiraceae,* more specifically, derived from bacteria of *Ruminococcus gnavus (R. gnavus*) belonging to the genus *Ruminococcus.* That is, in the present invention, the target of the pharmaceutical composition of the present invention may be either IbpA protein (SEQ ID NO: 1) or IbpB protein (SEQ ID NO: 2) or a variant thereof.

It is preferable that the substance that binds to an Ibp protein of a bacterium of the family *Lachnospiraceae* or its variant binds to the structure of the Ibp protein or its variant involved in the binding between the Ibp protein or its variant and IgE. Examples of the structure of the Ibp protein include, but not limited to, a protein of a repeat region comprising the amino acid sequence of amino acid Nos. 111 to 378 of SEQ ID NO: 1 or amino acid Nos. 126 to 393 of SEQ ID NO: 2 and a protein of C-terminal region comprising the amino acid sequence of amino acid Nos. 379 to 574 of SEQ ID NO: 1 or amino acid Nos. 394 to 592 of SEQ ID NO: 2.

The substance that binds to the Ibp protein or its variant may be a substance that can bind to a protein having an amino acid sequence comprising substitution, insertion, or deletion of one or several amino acids in the amino acid sequence of the above repeat region or C-terminal region; or a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB), or a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB). The substance of the present invention that binds to the Ibp protein of a bacterium of the family *Lachnospiraceae* or its variant can also bind to the modified amino acid sequences and thereby can also function, as a pharmaceutical composition, on an allergy occurring due to a mutated Ibp protein.

Based on the above findings, the present invention can provide, as a second embodiment, a vaccine composition for preventing or treating an allergy, comprising the full-length or a portion of the Ibp protein of a bacterium of the family *Lachnospiraceae* or its variant as an immunogen.

The vaccine composition in this embodiment of the present invention is aimed at preventing or treating allergies by producing an antibody to the full-length or a portion of the Ibp protein of a bacterium of the family *Lachnospiraceae* or its variant in a living body to which the vaccine composition is administered and by inhibiting the nonspecific binding between the Ibp protein or its variant and IgE in vivo by the antibody. That is, the present invention is aimed at preventing or treating allergies by producing the substance that binds to an Ibp protein of a bacterium of the family *Lachnospiraceae* or its variant in the first embodiment of the present invention in vivo.

Here, the Ibp protein that is used as an example of the active ingredient of the vaccine composition is derived from a bacterium of the family *Lachnospiraceae,* specifically, a bacterium of genus *Ruminococcus* and preferably *R. gnavus.*

The Ibp protein from *R. gnavus* mentioned as an example of the active ingredient of the vaccine composition of the present invention includes two homologs, IbpA protein (SEQ ID NO: 1) and IbpB protein (SEQ ID NO: 2), and the Ibp protein that is used as an ingredient of the vaccine may be any of them. The active ingredient of the vaccine composition of the present invention may be a variant of the Ibp protein (SEQ ID NO: 1 or SEQ ID NO: 2). Examples of the variant include a protein having an amino acid sequence comprising substitution, insertion, or deletion of one or several amino acids in the amino acid sequence of IbpA protein (SEQ ID NO: 1) or IbpB protein (SEQ ID NO: 2); a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB), and a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB).

The active ingredient of the vaccine composition may be the full-length or a portion of the Ibp protein or its variant. In the case where a portion of the Ibp protein or its variant is used as an ingredient of the vaccine, the antibody that is produced in vivo against a portion of the Ibp protein or its variant is required to inhibit nonspecific binding between the Ibp protein or its variant and IgE in vivo (that is, the antibody produced in vivo competes with IgE). Accordingly, the portion of the Ibp protein or its variant that can be used as the ingredient of the vaccine is preferably a protein of a repeat region comprising the amino acid sequence of amino acid Nos. 111 to 378 of SEQ ID NO: 1 or amino acid Nos. 126 to 393 of SEQ ID NO: 2 or a protein of C-terminal region comprising the amino acid sequence of amino acid Nos. 379 to 574 of SEQ ID NO: 1 or amino acid Nos. 394 to 592 of SEQ ID NO: 2.

The Ibp protein or its variant that is used as the ingredient of the vaccine composition is characterized in producing an antibody other than IgE in a human body to which the vaccine composition is administered. As described above, since it has been shown that IgE and the Ibp protein from a bacterium of the family *Lachnospiraceae* such as a bacterium of genus *Ruminococcus* are nonspecifically bind in vivo to occur an allergy, as a result, it is necessary that administration of the vaccine composition does not result in in vivo production of IgE against the Ibp protein or its variant. Accordingly, the vaccine composition of the present invention, when administered, preferably produces IgG, IgA, IgM, or IgD that binds to the Ibp protein of a bacterium of the family *Lachnospiraceae* such as *R. gnavus* or its variant, and more preferably produces IgG, IgM, or IgA.

The antibody that is produced in vivo as a consequence of administration of the vaccine composition preferably can bind to not only the Ibp protein (IbpA protein (SEQ ID NO: 1) or IbpB protein (SEQ ID NO: 2)) from *R. gnavus* but also a variant protein thereof, i.e., a variant protein having an amino acid sequence comprising substitution, insertion, or deletion of one or several amino acids in the amino acid sequences of either the above Ibp proteins; or a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB), or a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB). The ability to bind to the variant proteins enables the vaccine composition of the present invention to exert an effect of preventing or treating the allergy, even if the Ibp protein contains a mutation.

In general, the class of Ig to be produced is determined, to some extent, by the administration route of the vaccine composition. Accordingly, when the vaccine of the present invention is administered, an administration route that hardly produces IgE in vivo can be selected. Examples of such administration route include, but not limited to, subcutaneous administration, intradermal administration, intramuscular administration, intravenous administration, and mucosal administration (oral or nasal administration).

Based on the above findings, the present invention can provide, as a third embodiment, a method for detecting the presence of a predisposition to developing an allergy in the serum derived from a subject. Specifically, as a new finding of the present invention, it was revealed that the Ibp protein of a bacterium of the family *Lachnospiraceae* or its variant, when present in vivo, nonspecifically binds to IgE and consequently develops an allergy. Therefore, it is possible to detect the presence of a predisposition to developing an allergy, by the production of histamine by mast cells based on the binding of the Ibp protein of a bacterium of the family *Lachnospiraceae* or its variant and IgE in the serum derived from a subject.

As a specific method, the presence of a predisposition to developing an allergy in a subject can be detected by the following steps:
(1) a step of detecting the Ibp protein or its variant in a living body-derived sample collected from a subject; and
(2) a step of associating the detection of the Ibp protein or its variant in the above (1) with the subject having a predisposition to developing an allergy.

In the step of (1) of this method, the Ibp protein or its variant in a living body-derived sample can be detected by a known method using an antibody to the Ibp protein or its variant. Examples of the known method for quantitative measurement of the Ibp protein or its variant include, but not limited to, an enzyme method (color development detection), an immunological method (ELISA color development), HPLC (fluorescence detection), and column separation fluorescence detection.

In this method, the living body-derived sample refers to a blood sample or a fecal sample. According to the findings of the present invention, the Ibp protein or its variant is produced by a bacterium of the family *Lachnospiraceae* in intestinal bacterial flora, which is absorbed into the body to enter the serum of the subject. Accordingly, it is possible to detect the presence of a predisposition to developing an allergy in a subject by detecting the inclusion of the Ibp protein or its variant in feces or the uptake of the Ibp protein or its variant in serum.

In this method, the "Ibp protein or its variant" as a detection target refers to any of:
- IbpA protein (SEQ ID NO: 1) or IbpB protein (SEQ ID NO: 2);
- a protein having an amino acid sequence comprising substitution, insertion, or deletion of one or several amino acids in the above amino acid sequences;
- a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB); and
- a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB).
The "Ibp protein or its variant" specified by these proteins nonspecifically binds to IgE in in vivo serum when absorbed into the living body and, as a result, becomes a predisposition that induces mast cell activation.

It is also possible to detect the presence of a predisposition to developing an allergy in the serum derived from a subject, by another specific method comprising the following steps:
(1) a step of culturing human mast cells in the presence of the serum derived from a subject and measuring the amount of histamine in a culture supernatant;
(2) a step of adding and reacting IgG, IgM, or IgA to the serum from the subject, and then culturing human mast cells in the presence of the serum and measuring the amount of histamine in a culture supernatant; and
(3) a step of associating the amount of histamine obtained in the above (1) being higher than the amount of histamine obtained in the above (2) with the presence of a predisposition to developing an allergy in the blood of the subject.

In the steps (1) and (2) of this method, the production of histamine from human mast cells can be detected by quantitatively measuring histamine released in the culture supernatant by a known method. Examples of the known method for quantitative measurement of histamine include, but not limited to, an enzyme method (color development detection), an immunological method (ELISA color development), HPLC (fluorescence detection), and column separation fluorescence detection.

In the step (2) of this method, the IgG, IgM, or IgA that is reacted in advance with the serum derived from a subject may be an antibody that specifically binds to the Ibp protein (an antibody that specifically binds to a portion of the Ibp protein as an epitope) or may be an antibody that nonspecifically binds to the Ibp protein (for example, an isotype control antibody).

When the amount of histamine measured in the step (1) is higher than the amount of histamine measured in the step (2), it means that the binding between the Ibp protein and IgE present in the serum of the subject is inhibited by IgG, IgM, or IgA that has been reacted in advance with serum to be added during culturing of the human mast cells in the step (2), and shows that a complexed product of the Ibp protein and IgE as an allergy predisposition is present in the serum of the subject.

Based on the above findings, the present invention can provide, as a fourth embodiment, a method for selecting an inhibitor against functional relations between IgE and the Ibp protein of a bacterium of the family *Lachnospiraceae* or its variant. Specifically, as a new finding of the present invention, it was revealed that the production of histamine by mast cells can be inhibited by inhibiting the binding between the Ibp protein of a bacterium of the family *Lachnospiraceae* or its variant and IgE. Therefore, it is possible to find a compound that can prevent or treat an allergy by searching for a substance that can inhibit the production of histamine by mast cells based on the binding of the Ibp protein of a bacterium of the family *Lachnospiraceae* or its variant and IgE.

Specifically, it is possible to obtain an inhibitor against functional relations between IgE and the Ibp protein of a bacterium of the family *Lachnospiraceae* or its variant by the following steps:
(1) a step of culturing mast cells in the presence of a test substance under conditions containing IgE and an Ibp protein of a bacterium of the family *Lachnospiraceae* or its variant and measuring the amount of histamine in a culture supernatant;
(2) a step of culturing mast cells in absence of the test substance under conditions containing IgE and the Ibp protein of a bacterium of the family *Lachnospiraceae* or its variant and measuring the amount of histamine in a culture supernatant; and
(3) a step of selecting the test substance as a substance having activity of inhibiting functional relations between IgE and the Ibp protein of a bacterium of the family *Lachnospiraceae* or its variant, when the amount of histamine measured in the above (1) is lower than the amount of histamine measured in the above (2).

In the method of this embodiment of the present invention, the test substance may be, but is not limited to, a low-molecular compound or may be an antibody. For example, it is possible to select a target substance by carrying out this method in high throughput using a low-molecular compound library.

In the steps (1) and (2) of this method, the production of histamine from human mast cells can be detected by quantitatively measuring histamine released in the culture supernatant by a known method.

The present invention will now be specifically illustrated by the Examples. The Examples shown below are not intended to limit the present invention in any way.

### Examples

### Example 1: R. gnavus Binding properties of antibody

*R. gnavus* is known to increase in the intestines of pediatric allergy patients to cause allergies. In this Example, in order to investigate the mechanism by which *R. gnavus* causes an allergy, nonspecific binding of *R. gnavus* with an antibody was examined.

As *R. gnavus* strains, two strains JCM 6515 and JCM 31371 were provided from Riken BioResource Research Center (BRC), Japan Collection of Microorganisms (JCM) (Tsukuba City, Ibaraki, Japan).

Antibodies which bind to the bacterium strains were used as follows:
Human IgE (abcam [ab65866], purified human IgE);
Human IgG (abcam [ab65866], human IgG isotype control);
Human IgA (ThermoFisher Scientific [12000C], purified human IgA) ;
Mouse IgA (ThermoFisher Scientific [12000C], mouse IgA isotype control);
Mouse IgG (Sigma [M5284], IgG1 isotype control from mouse myeloma);
Mouse IgM (Sigma [M5170-1MG], IgM from lambda mouse myeloma); Mouse IgE (BD [554118], purified mouse IgE isotype control);
Rat IgG (ThermoFisher Scientific [02-9602], rat IgG isotype control);
Rabbit IgG (ThermoFisher Scientific [02-6102], rabbit IgG isotype control); and
Goat IgG (R&D [AB-108-C], goat IgG control).

The binding between a bacterium strain and an antibody was examined by flow cytometry. First, after blocking with 2% BSA/PBS, each of the above-mentioned antibodies (primary antibodies) derived from various species was added to 1 × 10⁷ *R*. *gnavus* cells to make the final concentration 10 µ(micro)g/mL, followed by reaction at 4°C for 30 minutes. After washing with PBS three times, labeled secondary antibody that recognize the corresponding primary antibody was added thereto, followed by reaction at 4°C for 30 minutes. After washing with PBS three times, the cells were resuspended in 2% BSA/PBS, followed by measurement with a flow cytometer. The following antibodies were used as labeled secondary antibodies that recognize the above-mentioned 10 types of primary antibodies:
Goat anti-human IgE cross-adsorbed secondary antibody, FITC (A-18800, Invitrogen);
Goat anti-human IgG (H+L) Cross-Adsorbed secondary antibody, Alexa Fluor^{™} 488 (A-11013, Invitrogen);
Rabbit anti-human IgA antibody-FITC complex (A80-103F, Bethyl Laboratories, Inc.);
Goat anti-mouse IgA-FITC (1040-02, SouthernBiotech);
Goat anti-mouse IgG (H+L) Cross-Adsorbed secondary antibody, Alexa Fluor 488 (A-11001, Invitrogen);
Alexa Fluor 488 anti-mouse IgM antibody (406521, BioLegend, Inc) ;
FITC rat anti-mouse IgE (553415, BD);
Goat anti-rat IgG (H+L) Cross-Adsorbed secondary antibody, Alexa Fluor 488 (A-11006, Invitrogen);
Goat anti-rabbit IgG (H+L) Cross-Adsorbed secondary antibody, Alexa Fluor 488 (A-11008, Invitrogen); and
Donkey anti-goat IgG (H+L) Cross-Adsorbed secondary antibody, Alexa Fluor 546(A-11056, Invitrogen).

The results are shown in Table 1. That is, it was demonstrated that regarding human antibodies, *R. gnavus* binds to all Ig types tested, IgE, IgG, and IgA, and regarding mouse antibodies, *R. gnavus* binds to IgG, IgM, and IgA but does not bind to IgE. However, it was found that when *R. gnavus* binds to various types of antibodies, the binding is nonspecific. Similarly, a bacteria of the genus *Ruminococcus* other than *R*. *gnavus* are also examined, and it was also found that each of bacteria belonging to the genus *Ruminococcus* nonspecifically binds to various types of antibodies, although the range thereof is not as wide as *R. gnavus.*

**[Table 1]**

| Bacteria name/ JCM No. | *Ruminococcus gnavus*/ 6515 | *Ruminococcus gnavus*/ 31371 |
|---|---|---|
| Human IgE | ○ | ○ |
| Human IgG | ○ | ○ |
| Human IgA | ○ | ○ |
| Mouse IgA | ○ | ○ |
| Mouse IgG | ○ | ○ |
| Mouse IgM | ○ | ○ |
| Mouse IgE | x | x |
| Rat IgG | ○ | ○ |
| Rabbit IgG | ○ | ○ |
| Goat IgG | x | x |

| | | |
|---|---|---|
| ○: bound, x: not bound | | |

### Example 2: Localization of Ibp protein in R. gnavus

In this Example, since it is known that IbpA and IbpB from *R. gnavus* bind to human and mouse IgA as superantigens (Non Patent Literature 2), localization of IbpA and IbpB from R. gnavus were examined.

### (2-1) Production of R. gnavus gene-disrupted strain

A plasmid for disrupting a gene in *R. gnavus* was produced as follows. The pJIR750ai plasmid (Merck) was cleaved with MluI in the intron sequence, and ligation was performed using an ermBRAM fragment (erythromycin resistant cassette containing retrotransposition-activated selectable marker (RAM)) produced by DNA synthesis (Eurofins Genomics) and Instant Sticky Ligase (New England Biolabs). The produced plasmid was used as a template for PCR to amplify a DNA fragment containing intron-ermBRAM-ltrA. The amplified DNA fragment was cleaved with HindIII and XhoI and ligated into the HindIII-XhoI site of pMTL83153 (University of Nottingham) to produce plasmid pNO201 for disrupting a gene. Intron sequences for disrupting ibpA and ibpB genes were predicted by TargeTron system (Merck). A modified intron sequence was amplified by PCR and was introduced into the HindIII-BsrGI site of pNO201 by ligation.

The produced plasmids for disrupting ibp gene were each introduced into *R. gnavus* by a conjugation method described below. Each plasmid was introduced into E. coli HB101/pRK24 by electroporation and was proliferated by culturing with shaking in 2 mL BHI medium (containing 10 p, (micro) g/mL tetracycline and 20 µ(micro)g/mL chloramphenicol) overnight. The obtained E. coli cells were suspended in 200 µ(micro)L of a culture medium of *R*. *gnavus* cultured in a BHIS medium (3.7% BHI (Brain Heart Infusion), 0.5% yeast extract, and 0.1% cysteine) overnight, and allowed to stand and cultured on a BHI agar medium at 37°C anaerobically for 8 hours. The bacterial cells on the BHI agar medium were suspended and collected in 1 mL of BHI, and were cultured on a BHIS-TCK (BHIS containing 10 p, (micro) g/mL thiamphenicol, 250 µ(micro)g/mL D-cycloserine, and 50 µ(micro)g/mL kanamycin) agar medium at 37°C anaerobically. Colonies that showed good growth were then cultured on a BHIS-Em (BHIS containing 20 µ(micro)g/mL erythromycin) agar medium to select colonies showing erythromycin resistance. Regarding the selected bacteria, gene disruption was confirmed by colony PCR using primers for IbpA (tgcttaacag gacttatggt tgtga (SEQ ID NO: 3) and tgtaacgcat ggaaatctcc ca (SEQ ID NO: 4)) and primers for IbpB (acaggtatca attgatacat catgtg (SEQ ID NO: 5) and tctctttctt acgggtctta gtatacca (SEQ ID NO: 6)).

### (2-2) Protein extraction of R. gnavus

*R. gnavus* cells (provided from Riken BioResource Research Center, JCM 6515T) cultured and proliferated anaerobically in a GAM medium (Nissui, 05422) were separated from the culture supernatant by centrifugation (10,000 x g for 1 minute). The obtained cells were suspended in 1 mL of PBS, to which 500 µ(micro)L of glass beads were added, and the cells were crushed using Shake Master Neo (at 1500 rpm for 10 minutes). After centrifugation (20,000 x g for 5 minutes), the supernatant collected was designated as a whole cell fraction. Separately, the cells collected after culturing were suspended in a 0.1% sodium deoxycholate solution, left to stand at 37°C for 30 minutes, and then heated at 95°C for 20 minutes, followed by centrifugation (20,000 x g, for 5 minutes) to collect the supernatant as a cell wall fraction (Cell wall). The amount of protein in each fragment was quantitatively measured using a BCA protein Assay (Thermo Fisher Scientific).

In comparison of the whole cell fraction (Whole cell) and the extracellular fraction (the above-described culture supernatant) (Extracellular), the collected bacterial cells were suspended in PBS containing 1 mg/mL lysozyme. The suspension was left to stand at room temperature for 5 minutes, and the equivalent amount of 2 x SDS sample buffer (0.125 mol/L Tris-HCl (pH 6.8), 4% SDS, 20% glycerol, 0.01% BPB, and 10% 2-mercaptoethanol) was added thereto, followed by heating at 95°C for 10 minutes. The obtained solution was designated as a whole cell fraction. Extracellular protein was collected using trichloroacetic acid (TCA). TCA (final concentration: 20%) was added to and well mixed with the culture supernatant, followed by centrifugation (20,000 × g for 5 minutes). The pellet was washed with ice-cooled acetone. The pellet was suspended in 1 × SDS sample buffer, and the suspension was designated as extracellular fraction protein.

Membrane vesicle (MV) was collected according to the method described in Obana, et al., 2017, Infect. Immun. *R*. *gnavus* was cultured in BHIS (brain heart infusion medium containing 0.5% yeast extract and 0.1% cysteine) overnight, and the culture supernatant was collected by centrifugation (20,000 x g, for 5 minutes). The supernatant was passed through a PVDF filter membrane with a pore size of 0.45 µ(micro)m, and then subjected to ultracentrifugation (150,000 x g, for 2 hours) to obtain a membrane vesicle pellet. The membrane vesicle pellet was suspended in a 45% iodixanol solution, and the membrane vesicle fragment was purified by density gradient centrifugation with iodixanol.

### (2-3) Western blotting

Each protein was separated by 4% to 15% gradient SDS-PAGE and was transferred on a PVDF membrane by a semi-dry method. As the primary antibody, 0.1 ng/mL human IgE isotype control antibody (Thermo Fisher Scientific) was used. The IgE bound to protein was labeled using a 1 : 10,000 diluted goat anti-human IgE cross-adsorbed secondary antibody, HRP (Thermo Fisher Scientific), and detected using ImmunoStar LD (FUJIFILM Wako Pure Chemical Corporation).

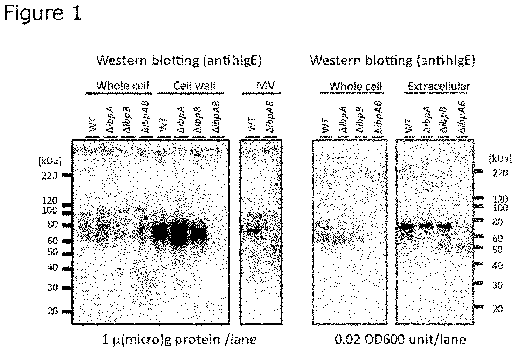

The results are shown in Figure 1. First, in order to investigate the intracellular localization of the Ibp protein, the proteins of *R. gnavus* were fractionated, and the Ibp protein contained in each fraction was analyzed. Since the band detected by Western blotting using IgE disappeared in Δ(delta)IbpA and Δ(delta)IbpB strains, it was confirmed that the protein detected here was the Ibp protein.

In the results shown on the left side of Figure 1, the comparison between the band intensities of the whole cell fraction and the cell wall fraction revealed that the Ibp protein is abundantly localized in the cell wall. This was consistent with the prediction that the Ibp protein contains a signal peptide (extracellular secretion signal) and an LPXTG motif (motif required for anchoring to cell wall).

Furthermore, the results shown on the left side of Figure 1 demonstrate that the Ibp protein is localized also in the membrane vesicles (MVs) produced by bacteria.

In order to investigate a possibility of extracellular secretion, the amounts of the Ibp protein in extracellular fractions were analyzed. As the results shown on the right side of Figure 1, it was revealed that the Ibp protein was abundantly present also extracellularly.

As described above, it was demonstrated that although the Ibp protein is present on and anchored to the cell wall, it is also extracellularly secreted in a free state.

### Example 3: Production of histamine from mast cells by allergen

In this Example, it was examined whether a substance derived from *R. gnavus* stimulates secretion of histamine in human mast cells culture strain or not.

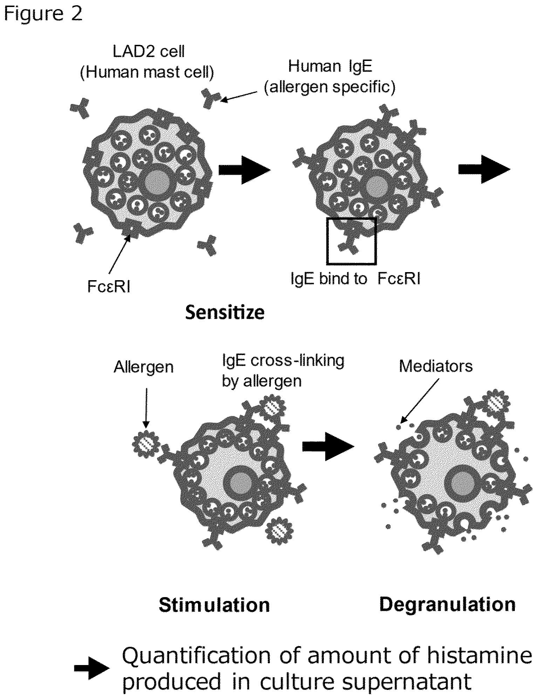

Regarding histamine release from activated mast cells, the reaction was confirmed by quantitatively measuring the amount of histamine released in the culture supernatant using LAD2 cells (human mast cell strain, National Institute of Allergy and Infectious Diseases, National Institute of Health), according to the schematic flow shown in Figure 2.

First, human mast cell culture strain LAD2 cells were seeded on a 96-well plate at a cell number of 1 × 10⁵ cells/well in StemPro-34 SFM (1 x) (Gibco) (L-glutamine (2 mM) (GIBCO), penicillin (100 U/mL)/streptomycin (100 µ(micro)g/mL) (GIBCO), recombinant human SCF (Peprotech, Inc) (100 ng/mL)). The cells were sensitized as a "step 1". Specifically, IgE was added to each well, followed by reaction at 37°C for 2 hours. As the IgE added to each well in this sensitization reaction, 1 µ(micro)g/mL of purified native human IgE protein (Abeam ab65866) was used for the test groups, and 10 µ(micro)g/mL of an anti-hapten 4-hydroxy-3-nitrophenylacetyl (NP)-specific human IgE (absolute antibody) was used for the positive control.

After the step 1, subsequently, as a "step 2", a step of stimulation was performed. Specifically, an allergen was added to sensitized cells in each well, which was stimulated by reaction at 37°C for 2 hours in the presence of 5% CO₂. The cells and the culture medium were collected and were centrifuged at 3,000 rpm at 4°C for 5 minutes to separate the cells and the culture supernatant from each other.

The allergens used in this stimulation reaction were as follows:
- Positive control: NP-BSA (LGC Biosearch Technologies) suspended in 100 µ(micro)L of a medium (final concentration: 100 ng/mL);
- Test group: control protein Rny suspended in 100 µ(micro)L of a medium (final concentration: 4.2 µ(micro)g/mL); and
- Test group: IbpA and IbpB proteins suspended in a 100 µ(micro)L of a medium (final concentration of IbpA: 2.4 µ(micro)g/mL, final concentration of IbpB: 2.1 µ(micro)g/mL, and total amount of Ibp proteins: 4.5 µ(micro)g/mL).
Combinations of the sensitizing substance and the stimulating substance are as shown in Table 2 below.

**[Table 2]**

| | Sensitizing substance | Stimulating substance |
|---|---|---|
| Untreated | - | - |
| Anti-NP hIgE | Anti-NP hIgE (0.1 µ(micro)g/ml) | - |
| Anti-NP hIgE + NP-BSA | Anti-NP hIgE (0.1 µ(micro)g/ml) | 10 ng NP-BSA |
| hIgE + Rny (control protein) | Purified hIgE (0.1 µ(micro)g/ml) | 420 ng Rny |
| hIgE + IbpA/IbpB mix | Purified hIgE (0.1 µ(micro)g/ml) | IbpA 240 ng + IbpB 217 ng mixture |

The obtained culture supernatant was then evaluated, as a step 3, for the amount of histamine degranulated and produced in the culture medium. Specifically, the amount of histamine produced in a culture supernatant was quantitatively measured using histamine EIA (Beckman Coulter, Inc.: IM2015).

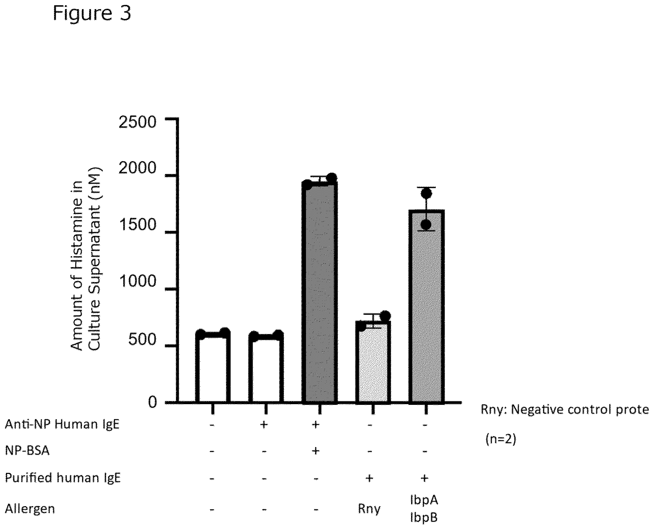

The results are shown in Figure 3. The graph of the figure displays the combinations in the order shown in Table 2 above, from the left. Sensitization with purified human IgE followed by stimulation with IbpA/IbpB derived from *R. gnavus* induced the production of histamine to the same extent as the positive control (sensitization with anti-NP-BSA and stimulation with NP-BSA).

These results revealed that a combination of sensitization with purified human IgE and stimulation with the Ibp protein (IbpA or IbpB) from *R. gnavus* promotes the production of histamine that causes allergy symptoms in the human mast cell culture strain.

### Example 4: Histamine production inhibition assay in LAD2 cells using human IgG

Since it was revealed in Example 3 that the combination of sensitization with purified human IgE and stimulation with the Ibp protein (IbpA or IbpB) from *R. gnavus* promotes production of histamine that causes allergy symptoms in human mast cell culture strain, in this Example, a method for inhibiting production of histamine by this mechanism has been examined.

First, human mast cell culture strain LAD2 cells were seeded on a 96-well plate at a cell number of 1 × 10⁵ cells/well in StemPro-34 SFM (1 x) (Gibco) (L-glutamine (2 mM) (GIBCO), penicillin (100 U/mL)/streptomycin (100 µ(micro)g/mL) (GIBCO, recombinant human SCF (Peprotech) (100 ng/mL)). The cells were sensitized with 10 µ(micro)g/mL of purified native human IgE protein (Abeam ab65866) (in the Figure, indicated by "+" in the column "Purified human IgE") or 100 µ(micro)g/mL of purified human IgG isotype control antibody (Invitrogen, 12000c) (in the Figure, indicated by "-" in the column of "Purified human IgE") for 2 hours at 37°C in the presence of 5% CO₂.

A hundred microliters of medium was put in a 1.5-mL tube, and IbpA and IbpB proteins (final concentration of IbpA: 2.4 µ(micro)g/mL, final concentration of IbpB: 2.1 µ(micro)g/mL, and total amount of the Ibp proteins: 4.5 µ(micro)g/mL) and human IgG isotype control antibody (final concentration: 5 µ(micro)g/mL) were provided to react during the step of sensitization at 37°C. In the step of stimulation, a sample of the IbpA and IbpB proteins (final concentration of IbpA: 2.4 µ(micro)g/mL, final concentration of IbpB: 2.1 µ(micro)g/mL, and total amount of the Ibp proteins: 4.5 µ(micro)g/mL) suspended in 100 µ(micro)L of a medium or a sample in which human IgG and the sample of the IbpA and IbpB proteins were reacted in advance was added, as an allergen, to the sensitized cells, followed by reaction for 2 hours at 37°C in the presence of 5% CO₂. The cells and the culture medium were collected from each well and were centrifuged at 3,000 rpm at 4°C for 5 minutes to separate the cells and the culture supernatant from each other.

The obtained culture supernatant was then evaluated, as a step 3, for the amount of histamine degranulated and produced in the culture medium. Specifically, the amount of histamine produced in a culture supernatant was quantitatively measured using histamine EIA (Beckman Coulter, Inc.: IM2015).

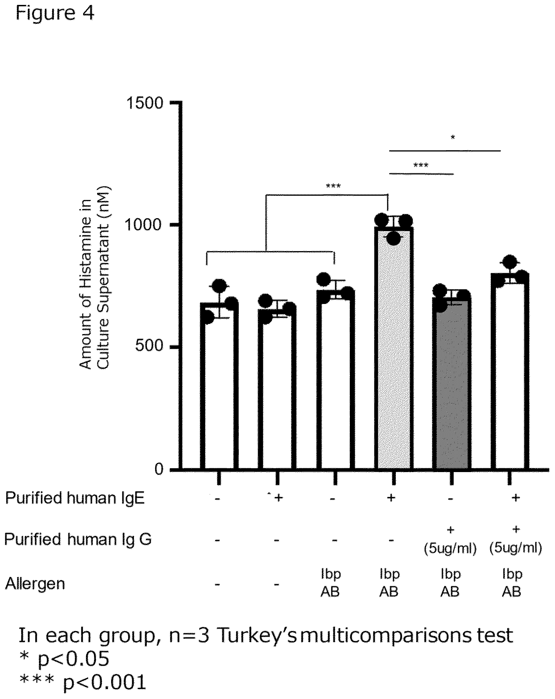

The results are shown in Figure 4. It was confirmed that a combination of sensitization with purified human IgE and stimulation with the Ibp protein from *R. gnavus* promotes the production of histamine that causes allergy symptoms in the human mast cell culture strain. In contrast, it was also revealed that when human mast cell culture strain sensitized with purified human IgE was stimulated by adding the Ibp protein from *R. gnavus* that has been reacted with human IgG in advance, binding between human IgE and the Ibp protein is inhibited to suppress the production of histamine (the rightmost column in the figure).

These results indicate the possibility that a molecule that inhibits the binding between human IgE and the Ibp protein from *R. gnavus* can suppress allergic response.

### Example 5: Examination of mode of binding between Ibp protein and IgE

Since the possibility that a molecule that inhibits the binding between human IgE and the Ibp protein from *R. gnavus* can suppress allergic response was indicated in Example 4, in this Example, the mode of binding between the Ibp protein and IgE was examined.

The genomic DNA of *R. gnavus* (NCBI Reference Sequence: NZ_CP027002.1) was used as a template to amplify fragments of the ibp gene in various lengths by PCR. The amplified DNA fragments were introduced into pET21b (Merck) by In-Fusion method (TAKARA Bio Inc.). In comparison between the amino acid sequences of IbpA and IbpB, although the sequences of 100 residues after about 30 residues of the N-terminal signal peptide were different from each other, the amino acid sequences in other regions are almost the same, i.e., there are a variable region (VR) of the N-terminal region of which the sequence differs between IbpA and IbpB and conserved repeat region consisting of 64 residues x 4 in both IbpA and IbpB sequences. Accordingly, the DNA fragments to be amplified were as follows:
- His6-ipbA: DNA encoding the sequence after the signal sequence of the IbpA protein (amino acids No. 30 to 574) with His6 tag at the N-terminus (primers: acatatgcat catcatcatc atcacgtgGC TGAAGCACCA GCAGAAGAG (SEQ ID NO: 7) and tgttagcagc cggatctcaA TTTTTCTTCT TATCTTCTTT CTTAGTATC (SEQ ID NO: 8));
- His6-ipbB: DNA encoding the sequence after the signal sequence of the IbpB protein (amino acids No. 30 to 592) with His6 tag at the N-terminus) (primers: tatacatatg catcatcatc atcatcacgt gGCAGAGCCA GTGGAAAAG (SEQ ID NO: 9) and tgttagcagc cggatctcaA TTTTTCTTCT TATCTTCTTT CTTAGTATC (SEQ ID NO: 8));
- His6-ipbA N100: DNA encoding 100 amino acids of the N-terminal side after the signal sequence of the IbpA protein (amino acids No. 30 to 129) with His6 tag at the N-terminus (primers: acatatgcat catcatcatc atcacgtgGC TGAAGCACCA GCAGAAGAG (SEQ ID NO: 7) and ttgttagcag ccggatctca TCCAGCTGCT TCATCGTAG (SEQ ID NO: 10));
- His6-ipbA N200: DNA encoding 200 amino acids of the N-terminal side after the signal sequence of the IbpA protein (amino acids No. 30 to 229) with His6 tag at the N-terminus (primers: acatatgcat catcatcatc atcacgtgGC TGAAGCACCA GCAGAAGAG (SEQ ID NO: 7) and ttgttagcag ccggatctca TTCAAGTGTA TATCCTTCTG GCA (SEQ ID NO: 11));
- His6-ipbA N349: DNA encoding 349 amino acids of the N-terminal side after the signal sequence of the IbpA protein (amino acids No. 30 to 378) with His6 tag at the N-terminus (primers: acatatgcat catcatcatc atcacgtgGC TGAAGCACCA GCAGAAGAG (SEQ ID NO: 7) and ttgttagcag ccggatctca TACAGAAACA TATACGTATC CATC (SEQ ID NO: 12));
- His6-ipbA N400: DNA encoding 400 amino acids of the N-terminal side after the signal sequence of the IbpA protein (amino acids No. 30 to 429) with His6 tag at the N-terminus (primers: acatatgcat catcatcatc atcacgtgGC TGAAGCACCA GCAGAAGAG (SEQ ID NO: 7) and ttgttagcag ccggatctca TCCTGTTGGA AGATTGAAGT C (SEQ ID NO: 13));
- His6-ipbA CR: DNA encoding the conservative repeat region of the IbpA protein (amino acids No. 110 to 378) with His6 tag at the N-terminus (primers: atcatcatca tcatcacgtg AAAGCACCTG TAGAAGAAGT ACAG (SEQ ID NO: 14) and ttgttagcag ccggatctca TACAGAAACA TATACGTATC CATC (SEQ ID NO: 12));
- His6-ipbA C200: DNA encoding 200 amino acids of the C-terminal side of the IbpA protein (amino acids No. 375 to 574) with His6 tag at the N-terminus (primers: tcatcatcat catcacgtgT ATGTTTCTGT AAAGAAAGAT GTAG (SEQ ID NO: 15) and tgttagcagc cggatctcaA TTTTTCTTCT TATCTTCTTT CTTAGTATC (SEQ ID NO: 8));
- His6-ipbB N200: DNA encoding 200 amino acids of the N-terminal side of the IbpB protein (amino acids No. 30 to 229) with His6 tag at the N-terminus (primers: tatacatatg catcatcatc atcatcacgt gGCAGAGCCA GTGGAAAAG (SEQ ID NO: 9) and ttgttagcag ccggatctca ATTTACACAC GATGTATCAA TTGA (SEQ ID NO: 16)); and
- His6-ipbB N364: DNA encoding 364 amino acids of the N-terminal side after the signal sequence of the IbpB protein (amino acids No. 30 to 393) with His6 tag at the N-terminus (primers: tatacatatg catcatcatc atcatcacgt gGCAGAGCCA GTGGAAAAG (SEQ ID NO: 9) and ttgttagcag ccggatctca TACAGAAACA TATACGTATC CATC (SEQ ID NO: 12)) (regarding IbpA, see Figure 5).

Ibp-expressing pET21 plasmids containing the produced DNA fragments, respectively, were introduced into NiCo21 (DE3) E. coli (New England Biolabs) and were cultured in an LB medium (containing 100 p, (micro) g/mL ampicillin) overnight, and each of the culture solutions was then inoculated at 1 : 100 into a fresh 2 x TY medium (containing 1.6% tryptone, 1.0% yeast extract, and 0.5% NaCl) containing 100 µ(micro)g/mL ampicillin, followed by culturing at 37°C at 220 rpm for 2 hours. Subsequently, isopropyl-β(beta)-thiogalactopyranoside (IPTG) was added thereto to be a final concentration of 1 mM, followed by further culturing for 4 hours.

Two hundred microliters of the culture solution were centrifuged (10,000 x g, for 1 minute) to collect the cells. The cell pellet was suspended in 1 × SDS sample buffer and heated at 95°C for 10 minutes to obtain a solution as an E. coli lysate sample containing a recombinant Ibp protein.

The E. coli lysate sample containing the recombinant Ibp protein was diluted 100 times and separated by SDS-PAGE. In each lane, 5 µ(micro)L of the 100-times diluted solution (equivalent to 0.05 µ(micro)L of the culture solution) was used.

The IgG binding properties were analyzed by Western blotting using 0.2 ng/mL human IgG isotype control antibody (Thermo Fisher Scientific) as the primary antibody. The primary antibody IgG bound to the protein on the membrane was labeled using a 1 : 5,000 diluted goat anti-human IgG (gamma chain) cross-adsorbed secondary antibody, HRP (Thermo Fisher Scientific), and was detected using ImmunoStar LD (FUJIFILM Wako Pure Chemical Corporation).

The IgA binding properties were analyzed by Western blotting using 0.2 ng/mL purified human IgA (Bethyl Laboratories, Inc.) as the primary antibody. The primary antibody IgA bound to the protein on the membrane was labeled using a 1 : 5,000 diluted goat anti-human IgA cross-adsorbed secondary antibody, HRP (Thermo Fisher Scientific), and was detected using ImmunoStar LD (FUJIFILM Wako Pure Chemical Corporation).

The IgE binding properties were analyzed by Western blotting using 0.2 ng/mL purified human IgE (Abeam ab65866) as the primary antibody. The primary antibody IgE bound to the protein on the membrane was labeled using a 1 : 5,000 diluted goat anti-human IgE cross-adsorbed secondary antibody, HRP (Thermo Fisher Scientific), and was detected using ImmunoStar LD (FUJIFILM Wako Pure Chemical Corporation).

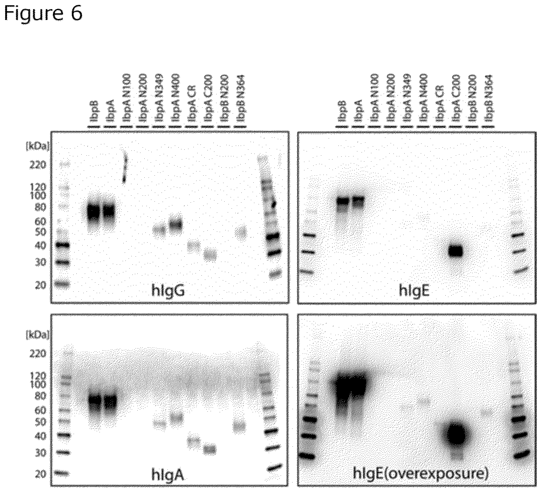

Figure 6 shows the results of Western blotting for the E. coli lysates. The full-length IbpA or IbpB exhibited the strongest IgG and IgA binding properties. In contrast, the repeat region and the C-terminal region did also bind to IgG and IgA, but exhibited the weak binding properties compared to that of the full-length (lanes: IbpA N349, IbpA N400, IbpA CR, and IbpA C200). The VR and the IbpA N100 and IbpA N200 slightly containing the repeat region in addition to VR, respectively, did not exhibit IgG and IgA binding properties at all.

Subsequently, the IgE binding properties were analyzed, and the strongest binding properties were observed in the C-terminal region (IbpA C200), unlike the IgG or IgA binding properties. IbpA N100 and IbpA N200 did not show IgE binding properties at all, and IbpA N349, IbpA N400, and IbpA CR containing the repeat region also showed almost no binding properties.

The above results revealed that, although high affinity for binding to IgG and IgA requires both the repeat region and the C-terminal region of the Ibp protein, the C-terminal region plays the main role for binding to IgE.

### Industrial Applicability

It was found that an Ibp protein from a bacterium of the family *Lachnospiraceae* is involved in development of allergies, and as a result, the present invention can provide a means for preventing or treating allergic reaction in vivo by administering a substance that binds to the Ibp protein of a bacterium of the family *Lachnospiraceae* (for example, a bacterium of genus *Ruminococcus*) or its variant to a living body. The present invention can also provide a vaccine composition containing the whole or a portion of the Ibp protein or its variant as an immunogen, wherein the vaccine composition is able to produce an antibody that binds to the Ibp protein or its variant in vivo upon administration to a living body.

## Claims

1. A pharmaceutical composition for treating or preventing an allergy, comprising a substance that binds to an Ibp protein of a bacterium of the family *Lachnospiraceae* or its variant.

2. The pharmaceutical composition according to Claim 1, wherein the Ibp protein or its variant is IbpA protein (SEQ ID NO: 1) or IbpB protein (SEQ ID NO: 2); a protein having an amino acid sequence comprising substitution, insertion, or deletion of one or several amino acids in these amino acid sequences; or a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB), or a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB).

3. The pharmaceutical composition according to Claim 1 or 2, wherein the substance that binds to an Ibp protein of a bacterium of the family *Lachnospiraceae* or its variant is an antibody or an antibody derivative.

4. The pharmaceutical composition according to Claim 1 or 2, wherein the substance that binds to an Ibp protein of a bacterium of the family *Lachnospiraceae* or its variant is IgG, IgM, or IgA.

5. The pharmaceutical composition according to Claim 1 or 2, wherein the bacterium of the family *Lachnospiraceae* is a bacterium of genus *Ruminococcus.*

6. The pharmaceutical composition according to Claim 5, wherein the bacterium of genus *Ruminococcus* is *Ruminococcus gnavus (R. gnavus*)*.*

7. The pharmaceutical composition according to Claim 1 or 2, wherein the substance that binds to an Ibp protein of a bacterium of the family *Lachnospiraceae* or its variant binds to a protein of a repeat region comprising the amino acid sequence of amino acid Nos. 111 to 378 of SEQ ID NO: 1 or amino acid Nos. 126 to 393 of SEQ ID NO: 2, a protein of C-terminal region comprising the amino acid sequence of amino acid Nos. 379 to 574 of SEQ ID NO: 1 or amino acid Nos. 394 to 592 of SEQ ID NO: 2, or a protein having an amino acid sequence comprising substitution, insertion, or deletion of one or several amino acids in the amino acid sequence of the repeat region or C-terminal region; or a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB), or a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB).

8. A vaccine composition for preventing or treating an allergy, comprising the full-length or a portion of an Ibp protein of a bacterium of the family *Lachnospiraceae* or its variant as an immunogen.

9. The vaccine composition according to Claim 8, wherein the Ibp protein or its variant is IbpA protein (SEQ ID NO: 1) or IbpB protein (SEQ ID NO: 2), or a protein having an amino acid sequence comprising substitution, insertion, or deletion of one or several amino acids in these amino acid sequences; or a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB), or a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB).

10. The vaccine composition according to Claim 8 or 9, wherein the bacterium *Lachnospiraceae* is a bacterium of genus *Ruminococcus.*

11. The vaccine composition according to Claim 10, wherein the bacterium of genus *Ruminococcus* is *Ruminococcus gnavus (R. gnavus*)*.*

12. The vaccine composition according to Claim 8 or 9, wherein the portion of the Ibp protein or its variant is a protein of a repeat region comprising the amino acid sequence of amino acid Nos. 111 to 378 of SEQ ID NO: 1 or amino acid Nos. 126 to 393 of SEQ ID NO: 2, a protein of C-terminal region comprising the amino acid sequence of amino acid Nos. 379 to 574 of SEQ ID NO: 1 or amino acid Nos. 394 to 592 of SEQ ID NO: 2, or a protein having an amino acid sequence comprising substitution, insertion, or deletion of one or several amino acids in the amino acid sequence of the repeat region or C-terminal region; or a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB), or a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB).

13. The vaccine composition according to Claim 8 or 9, wherein when the vaccine composition is administered, IgG, IgM, or IgA is produced in vivo.

14. The vaccine composition according to Claim 8 or 9, wherein IgG, IgM, or IgA that binds to the Ibp protein of a bacterium of the family *Lachnospiraceae* or its variant is produced in a human body to which the vaccine composition is administered.

15. The vaccine composition according to Claim 8 or 9, wherein the vaccine composition is administered by subcutaneous administration, intradermal administration, intramuscular administration, intravenous administration, or mucosal membrane administration.

16. A method for detecting the presence of a predisposition to developing an allergy in a subject, comprising:
(1) a step of detecting the Ibp protein or its variant in a living body-derived sample collected from a subject; and
(2) a step of associating the detection of the Ibp protein or its variant in the above (1) with that the subject has a predisposition to developing an allergy.

17. The method according to Claim 16, wherein the living body-derived sample is a blood sample or a fecal sample.

18. The method according to Claim 16 or 17, wherein the Ibp protein or its variant is IbpA protein (SEQ ID NO: 1) or IbpB protein (SEQ ID NO: 2), or a protein having an amino acid sequence comprising substitution, insertion, or deletion of one or several amino acids in these amino acid sequences; or a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB), or a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB).

19. A method for detecting the presence of a predisposition to developing an allergy in the serum derived from a subject, comprising:
(1) a step of culturing human mast cells in the presence of the serum derived from a subject and measuring the amount of histamine in a culture supernatant;
(2) a step of adding and reacting IgG, IgM, or IgA to the serum from the subject, and then culturing human mast cells in the presence of the serum and measuring the amount of histamine in a culture supernatant; and
(3) a step of associating the amount of histamine obtained in the above (1) being higher than the amount of histamine obtained in the above (2) with the presence of a predisposition to developing an allergy in the blood of the subject.

20. A method for selecting an inhibitor against functional relations between IgE and an Ibp protein of a bacterium of the family *Lachnospiraceae* or its variant, comprising:
(1) a step of culturing mast cells in the presence of a test substance under conditions containing IgE and an Ibp protein of a bacterium of the family *Lachnospiraceae* or its variant and measuring the amount of histamine in a culture supernatant;
(2) a step of culturing mast cells in absence of the test substance under conditions containing IgE and the Ibp protein of a bacterium of the family *Lachnospiraceae* or its variant and measuring the amount of histamine in a culture supernatant; and
(3) a step of selecting the test substance as a substance having an activity of inhibiting functional relations between IgE and the Ibp protein of a bacterium of the family *Lachnospiraceae* or its variant, when the amount of histamine measured in the above (1) is lower than the amount of histamine measured in the above (2).

21. The method according to Claim 20, wherein the Ibp protein or its variant is IbpA protein (SEQ ID NO: 1) or IbpB protein (SEQ ID NO: 2), or a protein having an amino acid sequence comprising substitution, insertion, or deletion of one or several amino acids in these amino acid sequences; or a protein having 50% or more amino acid sequence identity with amino acid Nos. 111 to 607 of SEQ ID NO: 1 (an amino acid sequence from the conservative portion of the repeat sequence to the C-terminus of IbpA) or with amino acid Nos. 126 to 625 of SEQ ID NO: 2 (an amino acid sequence from the conservative portion of the repeat sequence to the C-terminus of IbpB), or a protein having 80% or more amino acid sequence identity with amino acid Nos. 379 to 607 of SEQ ID NO: 1 (an amino acid sequence of the C-terminal region comprising the Ig-binding site of IbpA) or with amino acid Nos. 394 to 625 of SEQ ID NO: 2 (an amino acid sequence of the C-terminal region comprising the Ig-binding site of IbpB).

22. The method according to Claim 20 or 21, wherein the bacterium of the family *Lachnospiraceae* is a bacterium of genus *Ruminococcus.*

23. The method according to Claim 22, wherein the bacterium of genus *Ruminococcus* is *Ruminococcus gnavus* (*R. gnavus*)*.*
